# FASCICULE DE BREVET EUROPEEN

(11) **EP 4 110 279 B1**
(45) Date de publication et mention de la délivrance du brevet: **27.03.2024**
(21) Numéro de dépôt: 21707997.9
(22) Date de dépôt: 26.02.2021
(51) Int. Cl.: A61K 8/37, A61K 8/60, A61Q 13/00, A61K 8/04, A61K 8/06

(54) **COMPOSITION PERMETTANT DE DISPERSER UN COMPOSÉ LIPOSOLUBLE DANS UNE PHASE AQUEUSE**
ZUSAMMENSETZUNG ZUM DISPERGIEREN EINER FETTLÖSLICHEN VERBINDUNG IN EINER WÄSSRIGEN PHASE
COMPOSITION FOR DISPERSING A LIPOSOLUBLE COMPOUND IN AN AQUEOUS PHASE

(30) Priorité: 27.02.2020 FR 2001957
(43) Date de publication de la demande: 04.01.2023
(73) Titulaire: Sensient Cosmetic Technologies, 95310 Saint Ouen L'Aumone (FR)
(72) Inventeur: GUYOT-FERRÉOL, Véronique, Marguerite Suzanne, 92250 LA GARENNE COLOMBES (FR); DEGOURNAY, Anthony, 60600 ETOUY (FR)
(74) Mandataire: Lavoix
(86) Numéro de dépôt international: PCT/EP2021/054870
(87) Numéro de publication internationale: WO 2021/170817

(56) Documents cités:
- EP-A1- 2 366 376
- EP-B1- 2 366 376
- FR-A1- 2 797 764
- FR-B1- 2 797 764

## Description

La présente invention concerne une composition dispersante permettant de disperser un composé liposoluble, en particulier un parfum, dans une phase aqueuse.

La présence de composés liposolubles dans des compositions aqueuses est répandue dans de nombreux domaines, notamment dans ceux de la parfumerie, des cosmétiques ou des produits d'entretien. Dans le cas de compositions de parfum, le(s) composé(s) liposoluble(s) est(sont) incorporé(s) à la composition aqueuse notamment grâce à l'alcool éthylique. Des compositions aqueuses comprenant des composés liposolubles existent aussi sous la forme d'émulsions huile-dans-l'eau, dans lesquelles le(s) composé(s) liposoluble(s) est(sont) dispersé(s) grâce à des tensioactifs, qui sont souvent des composés éthoxylés.

Cependant, le marché de la parfumerie, des cosmétiques ou des produits d'entretien tend à s'orienter vers des compositions comprenant des composés plus respectueux de l'environnement, issus de la biomasse et/ou plus sûrs pour la santé. Or, l'alcool éthylique, comme les tensioactifs actuellement utilisés, ne répondent pas à ces critères. Dans ce contexte, le document EP2366376B1 est pertinent.

Par ailleurs, pour un grand nombre de compositions aqueuses comprenant au moins un composé liposoluble, un aspect transparent est requis.

Il existe donc un besoin de nouvelles compositions permettant de disperser un composé liposoluble dans une phase aqueuse.

Il existe également un besoin de nouvelles compositions permettant d'obtenir une émulsion transparente comprenant au moins un composé liposoluble.

Un des buts de la présente invention est donc de proposer une composition dispersante à base de composés naturels, biodégradables et/ou issus de biomasses renouvelables, qui permet de disperser au moins un composé liposoluble dans une composition aqueuse.

Un autre but de la présente invention est de proposer une composition dispersante permettant d'obtenir une émulsion transparente comprenant au moins un composé liposoluble, avec un ratio massique composition dispersante/composé liposoluble le plus faible possible. On cherche en effet à minimiser la quantité de composition dispersante au sein de l'émulsion, afin de limiter les coûts et/ou pour éviter les éventuels effets secondaires.

A cet effet, l'invention a pour objet une composition dispersante comprenant :
- au moins un polyglycoside d'alkyle, et
- un diester de formule (B) 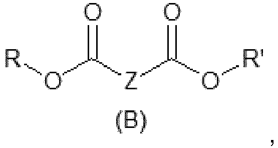
dans laquelle R et R', identiques ou différents, représentent un groupement alkyle, linéaire ou ramifié, comprenant de 1 à 10 atomes de carbone, et Z représente un groupement de formule -(CₙH₂ₙ)-, linéaire ou ramifié, n étant un nombre entier de 1 à 10, ladite composition dispersante étant telle que le ratio massique de la proportion massique de polyglycoside d'alkyle par rapport à la proportion massique de diester de formule (B) est de 7,4 à 18,0.

L'invention a également pour objet une émulsion comprenant
- une phase aqueuse, et
- une phase dispersée comprenant au moins un composé liposoluble ayant une valeur de logP strictement supérieure à 0, l'émulsion comprenant une composition dispersante selon l'invention.

L'invention concerne également l'utilisation de la composition dispersante selon l'invention pour améliorer la transparence d'une émulsion comprenant une phase aqueuse et une phase dispersée comprenant au moins un composé liposoluble ayant une valeur de logP strictement supérieure à 0.

L'invention concerne également l'utilisation de la composition dispersante selon l'invention pour disperser un composé liposoluble ayant une valeur de logP strictement supérieure à 0 dans une composition aqueuse.

Les inventeurs ont constaté qu'une composition dispersante comprenant l'association d'au moins un polyglycoside d'alkyle et d'un diester de formule (B) permet de disperser au moins un composé liposoluble dans une phase aqueuse et d'obtenir une émulsion transparente à un ratio massique de la somme des masses de polyglycoside d'alkyle et du diester de formule (B) par rapport à la masse du(des) composé(s) liposoluble(s) plus faible que le ratio massique :
- de la masse de polyglycoside d'alkyle par rapport à la masse du(des) composé(s) liposoluble(s) d'une composition exempte de diester de formule (B), et
- de la masse de diester de formule (B) par rapport à la masse du(des) composé(s) liposoluble(s) d'une composition exempte de polyglycoside d'alkyle.

Par « masse du(des) composé(s) liposoluble(s) », on entend la masse du composé liposoluble quand il n'y en a qu'un seul, ou la somme des masses des composés liposolubles quand il y en a plusieurs.

Avantageusement, afin d'obtenir une émulsion transparente en dispersant un composé liposoluble au sein d'une phase aqueuse, il est donc possible d'utiliser une proportion massique plus faible de composition dispersante selon l'invention que :
- la proportion massique de polyglycoside d'alkyle qui serait requise si ce dispersant était utilisé seul, et
- la proportion massique de diester de formule (B) qui serait requise si ce dispersant était utilisé seul.

De manière surprenante, les inventeurs ont découvert qu'il existe un effet de synergie entre le(les) polyglycoside(s) d'alkyle et le diester de formule (B) en ce qui concerne la dispersion d'au moins un composé liposoluble dans une phase aqueuse.

Dans la présente demande, les composés sont dénommés par leur dénomination INCI.

Un polyglycoside d'alkyle correspond à un polymère comprenant une chaîne polyglycosidique comprenant des unités glycoside dans laquelle au moins une fonction hydroxyle d'au moins une des unités glycoside est O-alkylée.

De préférence, la chaîne polyglycosidique comprend (voire est constituée) d'unités glucose et/ou pentose. Un polyglycoside d'alkyle est notamment un polyglucoside d'alkyle ou un polypentoside d'alkyle.

De préférence, le polyglycoside d'alkyle est un polyglucoside d'alkyle.

De préférence, le polyglycoside d'alkyle est un polyglycoside d'alkyle en C4-C14, de préférence en C8-C10. Le polyglycoside d'alkyle comprend alors une chaîne polyglycosidique dans laquelle au moins une fonction hydroxyle d'au moins une des unités glycoside est O-alkylée par un alkyle, linéaire ou ramifié, comprenant de 4 à 14 atomes de carbone, de préférence de 8 à 10 atomes de carbone.

Dans la présente demande, l'expression « de X à Y » signifie « est supérieur ou égal à X et est inférieur ou égal à Y ».

Selon un mode de réalisation préféré, le polyglycoside d'alkyle est du caprylyl/capryl wheat bran/straw glycosides, du coco-glucoside or caprylyl/capryl glucoside ou un mélange de ceux-ci, de préférence du coco-glucoside or caprylyl/capryl glucoside.

Les caprylyl/capryl wheat bran/straw glycosides sont la dénomination INCI correspondant au produit obtenu par la glycosylation entre un mélange d'alcools caprylique et caprique, et les monosaccharides dérivés de l'hydrolyse du son de blé et de la paille de blé.

Ci-après, le polyglycoside d'alkyle sera nommé indifféremment « polyglycoside d'alkyle » ou « dispersant A ».

De préférence, dans le diester de formule (B), le groupement Z est un groupement alkyle linéaire de formule -(CH₂)ₙ-.

Dans la formule (B), n est un entier de 1 à 10, préférentiellement n est un entier de 1 à 8, avantageusement n est un entier de 1 à 4.

Préférentiellement, dans le diester de formule (B), n est un entier égal à 1 ou 4, avantageusement n est égal à 4.

De préférence, dans la formule (B), R et R', identiques ou différents, représentent un groupement alkyle, linéaire ou ramifié, comprenant de 1 à 5 atomes de carbone, de préférence comprenant 2 ou 3 atomes de carbone.

De préférence, dans la formule (B), R et R' sont identiques et représentent un groupement isopropyle ou un groupement éthyle.

Avantageusement, le diester de formule (B) est choisi parmi le diisopropyl adipate, le malonate de diisopropyle et le diethyl adipate.

Le diisopropyl adipate est la dénomination INCI correspondant au composé correspondant au numéro CAS 6938-94-9. Le diisopropyl adipate est également connu sous la dénomination « isoadipate », « ester 1,6-bis(1-méthyléthyl) d'acide hexanedioique » ou « hexanedioate de dipropan-2-yle ».

Le malonate de diisopropyle correspond au composé de numéro CAS 13195-64-7.

Le diethyl adipate est la dénomination INCI correspondant au composé correspondant au numéro CAS 627-93-0.

Selon un mode de réalisation préféré, le diester de formule (B) est le diisopropyl adipate.

Dans un mode de réalisation particulièrement préféré, le polyglycoside d'alkyle est du coco-glucoside or caprylyl/capryl glucoside et le diester de formule (B) est choisi parmi le diisopropyl adipate, le malonate de diisopropyle et le diethyl adipate, plus préférentiellement le polyglycoside d'alkyle est du coco-glucoside or caprylyl/capryl glucoside et le diester de formule (B) est le diisopropyl adipate.

Ci-après, le diester de formule (B) sera nommé indifféremment « diester de formule (B) » ou « dispersant B ».

Sauf mention contraire, les proportions massiques en dispersant sont par rapport à la masse totale de la composition dispersante. Par exemple, la proportion massique de dispersant A est par rapport à la masse totale de la composition dispersante.

Le ratio massique A/B est la proportion massique de A par rapport à la proportion massique de B.

De préférence, dans la composition dispersante, le ratio massique A/B de la proportion massique de dispersant A par rapport à la proportion massique de dispersant B est de 9,0 à 15,0, de préférence de 9,5 à 15,0, préférentiellement de 9,5 à 13,0, plus préférentiellement de 8 à 13, avantageusement de 10,0 à 10,5.

Selon un mode de réalisation, la composition dispersante comprend en outre du triethyl citrate, le ratio massique de la proportion massique de polyglycoside d'alkyle par rapport à la proportion massique de triethyl citrate étant de 66,0 à 90,0.

Le triethyl citrate est la dénomination INCI correspondant au citrate de triéthyle selon la dénomination française (numéro CAS 77-93-0).

Dans la présente demande, le triethyl citrate est nommé indifféremment « triethyl citrate » ou « dispersant C ».

De préférence, le ratio massique A/C de la proportion massique de dispersant A par rapport à la proportion massique de dispersant C est de 66,0 à 80,0, de préférence de 70,0 à 80,0, avantageusement de 70,0 à 75,0.

La présence de triethyl citrate dans la composition dispersante exacerbe l'effet de synergie entre le polyglycoside d'alkyle et le diester de formule (B) en ce qui concerne la dispersion d'au moins un composé liposoluble dans une phase aqueuse.

De préférence, le ratio massique de la proportion massique de diester de formule (B) par rapport à la proportion massique de triethyl citrate est de 3,2 à 8,0.

De préférence, le ratio massique B/C de la proportion massique de dispersant B par rapport à la proportion massique de dispersant C est de 6,5 à 7,5, de préférence de 6,8 à 7,2, de manière avantageuse est égal à 7,0.

De telles valeurs de ratio massique B/C permettent d'améliorer les performances de la composition dispersante en ce qui concerne la dispersion d'au moins un composé liposoluble dans une solution aqueuse. De telles valeurs de ratio massique B/C permettent de diminuer le ratio massique dispersants/composé liposoluble utilisé dans l'émulsion tout en obtenant une émulsion transparente.

Dans une composition dispersante préférée :
- le ratio massique de la proportion massique de dispersant A par rapport à la proportion massique de dispersant B est de 7,4 à 18,0,
- le ratio massique de la proportion massique de dispersant A par rapport à la proportion massique de dispersant C est de 66,0 à 90,0, et
- le ratio massique de la proportion massique de dispersant B par rapport à la proportion massique de dispersant C est de 3,2 à 8,0.

Dans une composition dispersante préférée :
- le ratio massique de la proportion massique de dispersant A par rapport à la proportion massique de dispersant B est de 10,0 à 10,5,
- le ratio massique de la proportion massique de dispersant A par rapport à la proportion massique de dispersant C est de 70,0 à 75,0, et
- le ratio massique de la proportion massique de dispersant B par rapport à la proportion massique de dispersant C est de 6,8 à 7,2.

Dans une composition dispersante préférée :
- le ratio massique de la proportion massique de dispersant A par rapport à la proportion massique de dispersant B est de 7,2/0,7,
- le ratio massique de la proportion massique de dispersant A par rapport à la proportion massique de dispersant C est de 7,2/0,1, et
- le ratio massique de la proportion massique de dispersant B par rapport à la proportion massique de dispersant C est de 0,7/0,1.

Avantageusement, la composition dispersante selon l'invention est naturelle selon la norme ISO 16128-2:2017.

Selon l'invention, une composition naturelle est une composition dont la proportion massique en extrait sec de composés naturels est supérieur ou égal à 50%.

Par composé naturel, on entend un composé présentant un indice d'origine naturelle supérieur ou égal à 1 selon la norme ISO 16128-2:2017.

Par exemple, le caprylyl/capryl wheat bran/straw glycosides présente généralement un indice d'origine naturelle de 99,6.

Le triethyl citrate présente un indice d'origine naturelle de 1.

En effet, le triethyl citrate est de préférence obtenu par estérification d'acide citrique et d'éthanol, l'acide citrique et l'éthanol étant obtenus par fermentation de matières premières glucidiques issues de biomasses renouvelables.

Une composition dispersante naturelle a un impact environnemental moins élevé.

L'invention a également pour objet une émulsion comprenant
- une phase aqueuse, et
- une phase dispersée comprenant au moins un composé liposoluble ayant une valeur de logP strictement supérieure à 0,
l'émulsion comprenant une composition dispersante selon l'invention.

Le logP, aussi appelé *Log Kow,* est une mesure de la solubilité différentielle de composés chimiques entre l'octanol et l'eau (coefficient de partage octanol/eau).

Le logP est égal au logarithme du rapport des concentrations de la substance étudiée dans l'octanol et dans l'eau. logP = log(C_{oct}/Cₑₐᵤ). Cette valeur permet d'appréhender le caractère hydrophile ou hydrophobe (liposoluble) d'une molécule. En effet, si le logP est positif, cela exprime le fait que la molécule considérée est plus soluble dans l'octanol que dans l'eau, ce qui reflète son caractère liposoluble, et inversement.

Selon l'invention, un composé liposoluble a une valeur de logP strictement supérieure à 0, de préférence supérieure ou égal à 1.

De manière avantageuse, l'émulsion selon l'invention est transparente.

En effet, les inventeurs ont découvert que la combinaison de polyglycoside d'alkyle avec un diester de formule (B) permet d'obtenir une émulsion comprenant au moins un composé liposoluble tout en étant transparente.

Par « transparente », on entend une émulsion dont la densité optique à 600 nanomètres (nm) et à 25°C est de 0,0 à 0,1.

Si la densité optique à 600 nm de l'émulsion est strictement supérieure à 0,1, l'émulsion est considérée comme étant trouble.

La densité optique à 600 nm de l'émulsion est déterminée en utilisant un spectrophotomètre Konica Minolta modèle CM-5 en mode transmission pour une longueur d'onde de 600 nm.

L'émulsion à mesurer est placée dans une cuve dédiée à cet effet. Grâce à un monochromateur, une lumière monochromatique de longueur d'onde 600 nm traverse l'émulsion contenu dans la cuve. La valeur de l'intensité transmise à travers l'échantillon fourni alors la donnée de densité optique.

De préférence, l'émulsion selon l'invention est transparente à des températures de 4°C à 45°C.

De préférence, le composé liposoluble est choisi dans la liste constituée des molécules odorantes, des parfums, des composés émollients, des filtres ultraviolets et des actifs cosmétiques.

Avantageusement, le composé liposoluble est choisi dans la liste constituée du menthyl lactate, de l'ethylhexyl salicylate, du phenethyl alcohol, du cis-tetrahydro-2-isobutyl-4-methylpyran-4-ol, du trans-tetrahydro-2-isobutyl-4-methylpyran-4-ol (Florosa, EC N° 405-040-6, CAS N° 63500-71-0), du benzyl acetate, du linalool, de l'Hedione^{®}, de la gamma-décalactone, de la dimethyl myrcetone, du Verdox^{®}, de l'hexyl cinnamal, de l'amyl salicylate et de l'Habanolide^{®}.

De préférence, dans l'émulsion, le ratio massique de la somme des proportions massiques de polyglycoside d'alkyle, de diester de formule (B), et de l'éventuel triethyl citrate, par rapport à la proportion massique de composé liposoluble, est inférieur ou égal à 25, de préférence inférieur ou égale à 20.

Les proportions massiques au sein de l'émulsion sont par rapport à la masse totale de l'émulsion.

Avantageusement, dans l'émulsion, le ratio massique « (A+B+éventuel C)/ composé liposoluble » de la somme des proportions massiques de dispersant A, de dispersant B, et de l'éventuel dispersant C, par rapport à la proportion massique de composé liposoluble, est de 3 à 20, de préférence de 3 à 15, avantageusement de 3 à 10.

De préférence, dans l'émulsion, la proportion massique du composé liposoluble est de 1% à 5%, de préférence de 2% à 5% par rapport à la masse totale de l'émulsion.

La présence de la composition dispersante dans l'émulsion permet d'obtenir une émulsion comprenant une proportion massique élevée de composé liposoluble tout en restant transparente.

Selon un mode de réalisation, la phase dispersée de l'émulsion est sous la forme de gouttelettes présentant un diamètre moyen tel que mesuré par diffraction dynamique de la lumière de 10 nanomètres à 20 nanomètres.

La taille des gouttelettes peut être mesurée par diffraction dynamique de la lumière (DLS). Une telle taille de gouttelettes de la phase dispersée de l'émulsion permet à l'émulsion d'être transparente car les tailles sont inférieures à la longueur d'onde de la lumière.

De préférence, l'émulsion selon l'invention est une composition cosmétique, une composition de produit de parfumerie ou une composition de produit d'entretien ménager.

L'invention a également pour objet l'utilisation de la composition dispersante selon l'invention pour améliorer la transparence d'une émulsion comprenant une phase aqueuse et une phase dispersée comprenant au moins un composé liposoluble ayant une valeur de logP strictement supérieure à 0.

L'invention a également pour objet l'utilisation de la composition dispersante selon l'invention pour disperser un composé liposoluble ayant une valeur de logP strictement supérieure à 0 dans une composition aqueuse.

D'autres avantages de la présente invention apparaîtront au vu des exemples illustratifs exposés ci-après.

### EXEMPLE 1 : Effet synergique des dispersants

Dans l'exemple 1, le dispersant A est du caprylyl/capryl wheat bran/straw glycosides, le dispersant B est du diisopropyl adipate et le dispersant C est du triethyl citrate.

La capacité des dispersants compris dans la composition à disperser des composés liposolubles dans une phase aqueuse a été évaluée en utilisant, à titre de composé liposoluble, l'un des trois parfums suivants : « Almond », « Floral Aquatic » et « Musky » (Sensient Fragrance) selon le protocole suivant :
1) le composé liposoluble et le(s) dispersant(s) sont mélangés. Selon les quantités engagées, ce mélange peut être fait soit à la spatule, soit sur un plateau d'agitation magnétique, soit sous agitation avec un mobile de type hélice, pale cadre, ancre...
2) de l'eau déminéralisée est progressivement ajoutée au mélange composé liposoluble-dispersant(s).

Ce procédé n'est pas limitatif à la préparation d'émulsion transparente selon l'invention.

La transparence de chaque émulsion obtenue a été déterminée par mesure de leur densité optique (DO 600) à la longueur d'onde 600 nm. Pour effectuer la mesure de densité optique des émulsions, un spectrophotomètre Konica Minolta modèle CM-5 est utilisé en mode transmission pour une longueur d'onde de 600 nm.

L'émulsion à mesurer est placée dans une cuve dédiée à cet effet. Grâce à un monochromateur, une lumière monochromatique de longueur d'onde 600 nm traverse l'émulsion contenue dans la cuve. La valeur de l'intensité transmise à travers l'échantillon fourni alors la valeur de densité optique.

Un échantillon est considéré transparent si sa densité optique est inférieure ou égale à 0,1.

Les quantités de chaque dispersant sont exprimées en proportion massique par rapport à la masse totale de l'émulsion obtenue. La quantité de parfum est exprimée en proportion massique par rapport à la masse totale de l'émulsion obtenue. Par exemple, l'émulsion E1 comprend 9 % en masse de dispersant A, 1% en masse de dispersant B, 1% en masse de parfum « Musky », le reste (89% massique) étant de l'eau.

Les émulsions comparatives sont désignées par « EC » et les émulsions selon l'invention sont désignées par « E ».

Ces protocoles sont valables pour tous les exemples ci-après.

| | Prop. massique Dispersant A (%) | Prop. massique Dispersant B (%) | Prop. massique Dispersant C (%) | Parfum et Prop. Massique (%) | Ratio massique dispersants (A+B+C)/parfum | Transparence (DO 600) |
|---|---|---|---|---|---|---|
| EC1 | 10 | - | - | Floral aquatic 1 | 10 | > 1 |
| EC2 | 15 | - | - | Floral aquatic 1 | 15 | 0,00 |
| EC3 | 10 | - | - | Musky 1 | 10 | > 1 |
| EC4 | 15 | - | - | Musky 1 | 15 | 0,00 |
| EC5 | - | 10 | - | Floral aquatic 1 | 10 | Pas d'émulsion |
| EC6 | - | 25 | - | Floral aquatic 1 | 25 | Pas d'émulsion |
| EC7 | - | 10 | - | Musky 1 | 10 | Pas d'émulsion |
| EC8 | - | 25 | - | Musky 1 | 25 | Pas d'émulsion |
| EC9 | - | - | 10 | Floral aquatic 1 | 10 | Pas d'émulsion |
| EC11 | - | - | 25 | Floral aquatic 1 | 25 | Pas d'émulsion |
| EC12 | - | - | 10 | Musky 1 | 10 | Pas d'émulsion |
| EC13 | - | - | 25 | Musky 1 | 25 | Pas d'émulsion |
| E1 | 9 | 1 | - | Musky 1 | 10 | 0,00 |
| E2 | 9 | 1 | - | Floral aquatic 1 | 10 | 0,003 |
| E3 | 7,2 | 0,7 | 0,1 | Floral aquatic 1 | 8 | 0,00 |
| E4 | 9,0 | 0,875 | 0,125 | Floral aquatic 1 | 10 | 0,00 |
| E5 | 7,2 | 0,7 | 0,1 | Musky 1 | 8 | 0,00 |
| E6 | 9,0 | 0,875 | 0,125 | Musky 1 | 10 | 0,00 |

Utilisés seuls, ni le dispersant B (diisopropyl adipate) ni le dispersant C (triethyl citrate) ne permettent d'obtenir une émulsion, quel que soit le ratio dispersant/parfum utilisé (10 ou 25) et quel que soit le parfum (émulsions EC5-EC9 et EC11-EC13).

Utilisé seul, le dispersant A (caprylyl/capryl wheat bran/straw glycosides) permet d'obtenir une émulsion transparente lorsqu'il est utilisé à proportion élevée par rapport à celle du parfum (ratio dispersant A/parfum de 15 - émulsions EC2 et EC4), mais l'émulsion n'est plus transparente quand sa proportion diminue (ratio dispersant A/parfum de 10 - émulsions EC1 et EC3).

Par contre, lorsque le dispersant A est combiné au dispersant B (émulsions E1 et E2), une émulsion transparente est obtenue pour un ratio dispersants A+B/parfum égal à 10.

Un effet de synergie est donc observé entre le dispersant A et le dispersant B (comparer EC1 avec E1 et EC3 avec E2).

Cet effet de synergie est exacerbé en utilisant une combinaison des 3 dispersants A+B+C. La combinaison des 3 dispersants A+B+C permet d'obtenir une émulsion transparente comprenant une proportion dispersants A+B+C/parfum plus faible (ratio dispersant A+B+C/parfum de 8/1 - émulsions E3 et E5) que dans le cas où seuls les dispersants A et B sont présents (ratio dispersant A+B/parfum de 10/1 - émulsions E1 et E2).

### EXEMPLE 2: Evaluation de l'influence des ratios massiques A/B, A/C et B/C sur les performances des compositions dispersantes

Pour toutes les émulsions de l'exemple 2, le dispersant A est du caprylyl/capryl wheat bran/straw glycosides, le dispersant B est du diisopropyl adipate et le dispersant C est du triethyl citrate.

Toutes les émulsions de l'exemple 2 comprennent 1% massique de parfum « Musky ».

| | Prop. massique Dispersan t A (%) | Prop. massique Dispersa nt B (%) | Prop. massique Dispersa nt C (%) | Ratio massiq ue A/B | Ratio massique A/C | Ratio massiq ue B/C | Ratio massique dispersan ts (A+B+C)/ parfum | Transpare nce (DO 600) |
|---|---|---|---|---|---|---|---|---|
| EC14 | 8,0 | 2,0 | - | 4 | - | - | 10 | >1 |
| EC15 | 8,5 | 1,5 | | 5,67 | - | - | 10 | >1 |
| EC16 | 8,7 | 1,3 | - | 6,69 | - | | 10 | >1 |
| EC17 | 8,8 | 1,2 | - | 7,33 | - | - | 10 | >1 |
| E7 | 8,9 | 1,1 | - | 8,09 | - | - | 10 | 0,004 |
| E8 | 9 | 1 | - | 9 | - | - | 10 | 0,00 |
| E9 | 9,2 | 0,8 | - | 11,5 | - | - | 10 | 0,002 |
| E10 | 9,4 | 0,6 | | 15,7 | - | - | 10 | 0,001 |
| EC16 | 9,5 | 0,5 | - | 19 | - | - | 10 | 0,124 |
| EC17 | 9,8 | 0,2 | - | 49 | - | - | 10 | 0,459 |
| E11 | 7,165 | 0,731 | 0,104 | 9,80 | 68,9 | 7 | 8 | 0,001 |
| E5 | 7,2 | 0,7 | 0,1 | 10,29 | 72,0 | 7 | 8 | 0,00 |
| E12 | 7,28 | 0,630 | 0,090 | 11,56 | 80,9 | 7 | 8 | 0,00 |
| E13 | 7,344 | 0,574 | 0,082 | 12,79 | 89,6 | 7 | 8 | 0,003 |
| EC18 | 7,574 | 0,320 | 0,105 | 23,67 | 72,1 | 3,05 | 8 | 0,52 |
| EC19 | 7,496 | 0,4 | 0,104 | 18,74 | 72,1 | 3,85 | 8 | 0,129 |
| E14 | 7,417 | 0,48 | 0,103 | 15,45 | 72 | 4,66 | 8 | 0,005 |

### EXEMPLE 3 : Evaluation de l'influence du ratio massique dispersants/composé liposoluble à proportion massique en composé liposoluble constante sur les performances des compositions dispersantes

Toutes les émulsions de l'exemple 3 ont été préparées à partir d'une composition dispersante comprenant les dispersants A, B et C dans les proportions massiques suivantes : 7,2/0,7/0,1, ou à partir d'une composition dispersante comparative comprenant uniquement le dispersant A .

Pour toutes les émulsions de l'exemple 3, le dispersant A est du caprylyl/capryl wheat bran/straw glycosides, le dispersant B est du diisopropyl adipate et le dispersant C est du triethyl citrate.

Une série de composés liposolubles a été testée, comprenant entre autres les trois parfums « Almond », « Floral Aquatic » et « Musky » utilisés dans les exemples 1 et 2, mais aussi les composés suivants (désignés par leur dénomination INCI ou leur dénomination commerciale) : Menthyl lactate (*Symrise*), Ethylhexyl salicylate (*DSM*), Phenethyl alcohol *(Sensient Fragance),* Florosa *(Sensient Fragance),* Benzyl acetate *(Sensient Fragance),* Linalool *(Sensient Fragance),* Hedione^{®} *(Sensient Fragance),* Gamma-décalactone *(Sensient Fragance),* Dimethyl myrcetone (*Sensient Fragance,* Verdox^{®} (*Sensient Fragance*), Hexyl cinnamal *(Sensient Fragance),* Amyl salicylate *(Sensient Fragance)* et Habanolide^{®} *(Sensient Fragance).*

Toutes les émulsions de l'exemple 3 comprennent 1% massique de composé liposoluble.

| Dispersant(s) | Composé liposoluble et Prop. Massique (%) | Ratio massique dispersant(s)/composé liposoluble | Transparence (DO 600) |
|---|---|---|---|
| A+B+C | Parfum Floral aquatic 1 | 8/1 | 0,00 |
| A | Parfum Floral aquatic 1 | 10/1 | > 1 |
| A+B+C | Parfum Musky 1 | 8/1 | 0,00 |
| A | Parfum Musky 1 | 10/1 | > 1 |
| A+B+C | Hedione ^{®} 1 | 15/1 | 0,00 |
| A | Hedione ^{®} 1 | 15/1 | 0,156 |
| A+B+C | Gamma decalactone 1 | 15/1 | 0,013 |
| A | Gamma decalactone 1 | 15/1 | 0,217 |
| A+B+C | Habanolide ^{®} 1 | 10/1 | 0,001 |
| A | Habanolide ^{®} 1 | 10/1 | > 1 |
| A+B+C | Parfum Almond 1 | 3/1 | 0,019 |
| A+B+C | Parfum Almond 1 | 5/1 | 0,00 |
| A+B+C | Menthyl lactate 1 | 19/1 | 0,00 |
| A+B+C | Ethylhexyl salicylate 1 | 20/1 | 0,00 |
| A+B+C | Phenethyl alcohol 1 | 10/1 | 0,021 |
| A+B+C | Florosa 1 | 5/1 | 0,002 |
| A+B+C | Benzyl acetate 1 | 20/1 | 0,003 |
| A+B+C | Linalool 1 | 20/1 | 0,009 |
| A+B+C | Dimethyl myrcetone 1 | 10/1 | 0,001 |
| A+B+C | Verdox ^{®} 1 | 10/1 | 0,002 |
| A+B+C | Hexyl cinnamal 1 | 15/1 | 0,00 |
| A+B+C | Amyl salicylate 1 | 15/1 | 0,004 |

La composition dispersante selon l'invention permet d'obtenir des émulsions transparentes comprenant une grande variété de composés liposolubles.

L'effet de synergie entre les dispersants A, B et C mentionné à l'exemple 1 est également observé avec ces autres composés liposolubles : par rapport à l'utilisation du dispersant A seul, l'utilisation d'une composition dispersante selon l'invention nécessite une moins grande quantité de dispersants pour obtenir une émulsion transparente comprenant un composé liposoluble. Autrement dit, pour certains ratios dispersant(s)/composé liposoluble, le dispersant A seul ne permet pas d'obtenir une émulsion transparente, contrairement à la composition dispersante selon l'invention.

### EXEMPLE 4 : Evaluation de l'influence de la proportion en composé liposoluble sur les performances des compositions dispersantes

Toutes les émulsions de l'exemple 4 ont été préparées à partir d'une composition dispersante comprenant les dispersants A, B et C dans les proportions massiques suivantes : 7,2/0,7/0,1.

Dans toutes les émulsions de l'exemple 4, le dispersant A est du caprylyl/capryl wheat bran/straw glycosides, le dispersant B est du diisopropyl adipate et le dispersant C est du triethyl citrate.

La proportion massique de composé liposoluble dans l'émulsion a été augmentée à 2%, 3% et 4%. La proportion massique en dispersants dans l'émulsion a également été progressivement augmentée afin de déterminer le ratio massique dispersants/composé liposoluble minimal permettant d'obtenir une émulsion transparente comprenant 2%, 3% ou 4% massique dudit composé liposoluble.

| Prop. massique de dispersants (A+B+C) (en %) | Composé liposoluble et Prop. Massique (%) | Ratio massique dispersants/composé liposoluble | Transparence (DO 600) |
|---|---|---|---|
| 3 | Parfum Almond 1 | 3/1 | 0,00 |
| 6 | Parfum Almond 2 | 6/2 = 3/1 | 0,03 |
| 8 | Parfum Floral aquatic 1 | 8/1 | 0,00 |
| 18 | Parfum Floral aquatic 2 | 18/2 = 9/1 | 0,002 |
| 28 | Parfum Floral aquatic 3 | 28/3 | 0,007 |
| 7 | Parfum Musky 1 | 7/1 | 0,001 |
| 8 | Parfum Musky 1 | 8/1 | 0,00 |
| 16 | Parfum Musky 2 | 16/2 | 0,08 |
| 28 | Parfum Musky 3 | 28/3 | 0,01 |
| 38 | Parfum Musky 4 | 38/4 | 0,006 |
| 19 | Menthyl lactate 1 | 19/1 | 0,00 |
| 36 | Menthyl lactate 2 | 36/2 | 0,00 |
| 57 | Menthyl lactate 3 | 57/3 | 0,00 |
| 76 | Menthyl lactate 4 | 76/4 | 0,00 |

Les résultats obtenus montrent que l'utilisation d'une composition dispersante selon l'invention permet d'obtenir des émulsions comprenant une proportion massique en composé liposoluble jusqu'à 4% massique qui sont transparentes.

### EXEMPLE 5 : Influence de la nature du diester de formule B

Dans l'exemple 5, le dispersant A est du caprylyl/capryl wheat bran/straw glycosides, le dispersant C est du triethyl citrate et le dispersant B est de formule suivante : 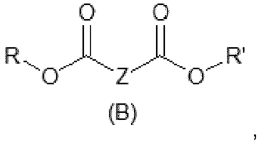 avec R et R', identiques ou différents, représentant un groupement alkyle, linéaire ou ramifié, comprenant de 1 à 10 atomes de carbone, et Z représentant un groupement de formule -(CₙH₂ₙ)-, linéaire ou ramifié, n étant un nombre entier de 1 à 10.

Toutes les émulsions de l'exemple 5 ont été préparées à partir d'une composition dispersante comprenant les dispersants A, B et C dans les proportions massiques suivantes : 7,2/0,7/0,1.

Toutes les émulsions de l'exemple 5 comprennent 1% massique de parfum « Musky », et un ratio massique (A+B+C)/parfum de 8/1.

| | Nature du dispersant B | Transparence (DO 600) |
|---|---|---|
| E15 | Diisopropyl adipate | 0,00 |
| E16 | Malonate de diisopropyle | 0,01 |
| E17 | Diethyl adipate | 0,01 |

## Revendications

1. Composition dispersante comprenant :
- au moins un polyglycoside d'alkyle, et
- un diester de formule (B) 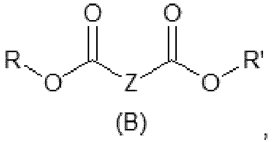
dans laquelle R et R', identiques ou différents, représentent un groupement alkyle, linéaire ou ramifié, comprenant de 1 à 10 atomes de carbone, et Z représente un groupement de formule -(CₙH₂ₙ)-, linéaire ou ramifié, n étant un nombre entier de 1 à 10,
ladite composition dispersante étant telle que le ratio massique de la proportion massique de polyglycoside d'alkyle par rapport à la proportion massique de diester de formule (B) est de 7,4 à 18,0.

2. Composition selon la revendication 1, dans laquelle le polyglycoside d'alkyle est un polyglycoside d'alkyle en C4-C14, de préférence en C8-C10.

3. Composition selon la revendication 1 ou 2, dans laquelle R et R', identiques ou différents, représentent un groupement alkyle, linéaire ou ramifié, comprenant de 1 à 5 atomes de carbone, de préférence comprenant 2 ou 3 atomes de carbone.

4. Composition dispersante selon l'une quelconque des revendications 1 à 3, comprenant en outre du triethyl citrate, le ratio massique de la proportion massique de polyglycoside d'alkyle par rapport à la proportion massique de triethyl citrate étant de 66,0 à 90,0.

5. Composition dispersante selon la revendication 4, dans laquelle le ratio massique de la proportion massique de diester de formule (B) par rapport à la proportion massique de triethyl citrate est de 3,2 à 8,0.

6. Emulsion comprenant :
- une phase aqueuse, et
- une phase dispersée comprenant au moins un composé liposoluble ayant une valeur de logP strictement supérieure à 0,
l'émulsion comprenant une composition dispersante selon l'une quelconque des revendications 1 à 5.

7. Emulsion selon la revendication 6, dans laquelle le ratio massique de la somme des proportions massiques de polyglycoside d'alkyle, de diester de formule (B), et de l'éventuel triethyl citrate, par rapport à la proportion massique de composé liposoluble, est inférieur ou égal à 25, de préférence inférieur ou égale à 20.

8. Emulsion selon la revendication 6 ou 7, dans laquelle la proportion massique du composé liposoluble est de 1% à 5%, de préférence de 2% à 5% par rapport à la masse totale de l'émulsion.

9. Emulsion selon l'une quelconque des revendications 6 à 8, dont la phase dispersée est sous la forme de gouttelettes présentant un diamètre moyen tel que mesuré par diffraction dynamique de la lumière de 10 nanomètres à 20 nanomètres.

10. Utilisation de la composition dispersante selon l'une quelconque des revendications 1 à 5 pour améliorer la transparence d'une émulsion comprenant une phase aqueuse et une phase dispersée comprenant au moins un composé liposoluble ayant une valeur de logP strictement supérieure à 0.

11. Utilisation de la composition dispersante selon l'une quelconque des revendications 1 à 5 pour disperser un composé liposoluble ayant une valeur de logP strictement supérieure à 0 dans une composition aqueuse.

## Patentansprüche

1. Dispergiermittelzusammensetzung, umfassend:
- mindestens ein Alkylpolyglykosid, und
- ein Diester der Formel (B) 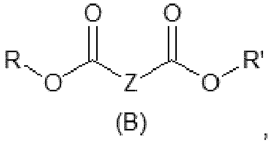
in der R und R', die gleichartig oder verschieden sind, eine geradkettige oder verzweigte Alkylgruppe mit 1 bis 10 Kohlenstoffatomen darstellen, und Z eine geradkettige oder verzweigte Gruppe der Formel -(CₙH₂ₙ)- darstellt, wobei n eine ganze Zahl von 1 bis 10 ist, wobei die Dispergiermittelzusammensetzung so beschaffen ist, dass das Massenverhältnis des Massenanteils des Alkylpolyglykosids zum Massenanteil des Diesters der Formel (B) 7,4 bis 18,0 beträgt.

2. Zusammensetzung nach Anspruch 1, wobei das Alkylpolyglykosid ein C4-C14-, vorzugsweise ein C8-C10-Alkylpolyglykosid ist.

3. Zusammensetzung nach Anspruch 1 oder 2, in der R und R', die gleich oder verschieden sind, eine geradkettige oder verzweigte Alkylgruppe mit 1 bis 5 Kohlenstoffatomen, vorzugsweise mit 2 oder 3 Kohlenstoffatomen, darstellen.

4. Dispergiermittelzusammensetzung nach einem der Ansprüche 1 bis 3, die zusätzlich Triethylcitrat enthält, wobei das Massenverhältnis des Massenanteils des Alkylpolyglykosids zum Massenanteil des Triethylcitrats 66,0 bis 90,0 beträgt.

5. Dispergiermittelzusammensetzung nach Anspruch 4, wobei das Massenverhältnis des Massenanteils des Diesters der Formel (B) zu dem Massenanteil des Triethylcitrats 3,2 bis 8,0 beträgt.

6. Emulsion, umfassend:
- eine wässrige Phase und
- eine dispergierte Phase, die mindestens eine fettlösliche Verbindung mit einem logP-Wert von strikt über 0 umfasst,
wobei die Emulsion eine Dispergiermittelzusammensetzung nach einem der Ansprüche 1 bis 5 umfasst.

7. Emulsion nach Anspruch 6, wobei das Massenverhältnis der Summe der Massenanteile von Alkylpolyglykosid, Diester der Formel (B) und gegebenenfalls Triethylcitrat, bezogen auf den Massenanteil der fettlöslichen Verbindung, kleiner oder gleich 25, vorzugsweise kleiner oder gleich 20, ist.

8. Emulsion nach Anspruch 6 oder 7, wobei der Massenanteil der fettlöslichen Verbindung 1 % bis 5 %, vorzugsweise 2 % bis 5 %, bezogen auf die Gesamtmasse der Emulsion, beträgt.

9. Emulsion nach einem der Ansprüche 6 bis 8, deren dispergierte Phase in Form von Tröpfchen mit einem mittleren Durchmesser, gemessen durch dynamische Lichtbeugung, von 10 Nanometern bis 20 Nanometern vorliegt.

10. Verwendung der Dispergiermittelzusammensetzung nach einem der Ansprüche 1 bis 5 zur Verbesserung der Transparenz einer Emulsion, die eine wässrige Phase und eine dispergierte Phase mit mindestens einer fettlöslichen Verbindung mit einem logP-Wert strikt über 0 umfasst.

11. Verwendung der Dispergiermittelzusammensetzung nach einem der Ansprüche 1 bis 5 zum Dispergieren einer fettlöslichen Verbindung mit einem logP-Wert von strikt über 0 in einer wässrigen Zusammensetzung.

## Claims

1. A dispersant composition comprising:
- at least one alkyl polyglucoside, and
- a diester of formula (B) 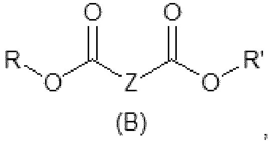
in which R and R', being the same or different, represent an alkyl group, linear or branched, comprising from 1 to 10 carbon atoms, and Z is a linear or branched group of formula -(CₙH₂ₙ)-, n being an integer from 1 to 10,
the dispersant composition being such that the mass ratio of the proportion by weight of alkyl polyglycoside relative to the proportion by weight of diester of formula (B) is from 7.4 to 18.0.

2. The composition according to claim 1, wherein the alkyl polyglycoside is a C4-C14, preferably C8-C10, alkyl polyglycoside.

3. The composition according to claim 1 or 2, in which R and R', which may be identical or different, represent a linear or branched alkyl group comprising from 1 to 5 carbon atoms, preferably comprising 2 or 3 carbon atoms.

4. The dispersant composition according to any one of claims 1 to 3, further comprising triethyl citrate, the mass ratio of the proportion by weight of alkyl polyglycoside to the proportion by weight of triethyl citrate being from 66.0 to 90.0.

5. The dispersant composition according to claim 4, wherein the mass ratio of the proportion by weight of diester of formula (B) to the proportion by weight of triethyl citrate is from 3.2 to 8.0.

6. An emulsion comprising:
- an aqueous phase, and
- a dispersed phase comprising at least one liposoluble compound with a logP value strictly greater than 0,
the emulsion comprising a dispersant composition according to any of claims 1 to 5.

7. The emulsion according to claim 6, wherein the mass ratio of the sum of the mass proportions of alkyl polyglycoside, diester of formula (B), and triethyl citrate if any, to the proportion by weight of liposoluble compound, is less than or equal to 25, preferably less than or equal to 20.

8. The emulsion according to claim 6 or 7, wherein the proportion by weight of the liposoluble compound is from 1% to 5%, preferably from 2% to 5%, based on the total weight of the emulsion.

9. The emulsion according to any one of claims 6 to 8, the dispersed phase of which is in the form of droplets having an average diameter as measured by dynamic light scattering of from 10 nanometers to 20 nanometers.

10. Use of the dispersant composition according to any one of claims 1 to 5 for improving the transparency of an emulsion comprising an aqueous phase and a dispersed phase comprising at least one liposoluble compound having a logP value strictly greater than 0.

11. Use of the dispersant composition according to any one of claims 1 to 5 for dispersing a liposoluble compound having a logP value strictly greater than 0 in an aqueous composition.
